# EUROPEAN PATENT APPLICATION

(11) **EP 3 088 021 A1**
(43) Date of publication of application: **02.11.2016**
(21) Application number: 15305640.3
(22) Date of filing: 27.04.2015
(51) Int. Cl.: A61M 5/14, A61M 5/145, A61M 5/172

(54) **INFUSION PUMP BASE MODULE WITH PLURALITY OF ATTACHABLE CONTROL MODULES**

(71) Applicant: Fresenius Vial SAS, 38590 Brézins (FR)
(72) Inventor: Couillaud, Frederic, 38500 Coublevie (FR); Ruton, Stephane, 38200 Vienne (FR); Marcoux, Sebastien, 38337 Rives (FR)
(74) Representative: Fresenius Kabi Deutschland GmbH

(57) **Abstract**

A base module (1,1') for controlling infusion pumps (14,3) is connected with at least one infusion pump for infusing liquids, in particular into a circulatory system of a patient. Therein, a plurality of module connectors (10) is provided, via each of which the base module (1, 1') is connectable with an external control module (2), wherein each of the module connectors (10) provides a module interface, wherein the base module (1, 1') is adapted to receive control information from a control module (2) via the module interface provided by the module connector (10) the control module (2) is connected to and to control at least one infusion pump (14,3) connected with the base module (1, 1') in dependence on the received control information, which derives from patient parameter sensors (25) connected to the respective control modules. In this way a base module is provided which allows to perform a plurality of infusion control functions via different connected control modules. A communication test method between control and base module is also disclosed.

## Description

The invention relates to a base module for controlling infusion pumps according to the preamble of claim 1, to a medical system and to a method for testing a medical function according to the preamble of claim 15.

A base module is operatively connected or connectable with at least one infusion pump. The infusion pump serves for infusing liquids, particularly in the circulatory system of a patient, wherein the liquids commonly comprise medication and/or nutrients. An infusion can be applied, e.g., intravenously, arterially, subcutaneously and/or epidurally. Base modules of this type are known to the applicant from practice.

The base module is adapted to control the infusion pump to perform a specific type of infusion. As an example, the infusion pump may be controlled to perform a continuous infusion (maintaining a substantially constant infusion rate, i.e., volume or mass of the infused liquid per unit of time), an intermittent infusion, a patient-controlled infusion and/or more complex types of infusion. In the latter, the infusion rate may particularly be controlled to follow a curve over the elapsed time. The curve may be predefined or may be calculated taking into account constant or variable patient-related information, such as age, gender, weight, blood pressure, heart rate, the concentration of substances in the blood, body temperature and the like. The base module may be adapted to provide a variety of different types of infusion or, more generally, different medical functions. In a given application, the appropriate medical function may be selected, which may be, for example, a specific type of infusion.

Infusions may be critical for the health and/or a physical recovery of the patient. It is, therefore, important to ensure the proper operation of the medical functions provided by the base module. Therefore, the base module is to be tested for proper operation, e.g., before its first use or after a modification such as a software update. Depending on how many different medical functions are provided by the base module, such tests can become time-consuming. This particularly holds when the base module provides complex medical functions. Such medical functions may provide a curve for an infusion rate over a given period of time, e.g. several hours or days. A test of the base module, in particular a base module providing a variety of medical functions, therefore, may take considerable time.

When a plurality of medical functions are provided by the base module, the individual medical functions may depend on each other, may be correlated with each other and/or may depend on common resources (e.g. hardware, software, parameter values and/or formulae). Thus, it may be necessary to test several or even all medical functions provided by the base module after an addition or a modification of at least one medical function. Such a test can be very time-consuming and may complicate a development of the base module.

Moreover, a plurality of N medical functions may be available in total. The base module may selectively be equipped with the necessary resources in order to provide one of, a selection of or all of the N available medical functions, depending on the need of a specific user. As a result, it may be necessary to perform at least one test for each possible combination of ≤ N medical functions. This may result in a total number of tests of the order of 2^{N}. The performance of such a large number of tests may be very time-consuming and labour-intensive. Since the tests need to be performed for ensuring a reliable function of the base module, it may become necessary to limit the number of available medical functions N, so that the number of necessary tests becomes reasonable.

It is an object of the instant invention to provide a base module and a method which allow to perform a plurality of medical functions while ensuring a reliable function of the base module with a reasonable test effort.

This object is achieved with a base module comprising the features of claim 1.

Accordingly, the base module comprises a plurality of (hardware) module connectors via each of which the base module is operatively connectable with an external control module. Therein, each of the module connectors provides a module interface and the base module is adapted to receive control information from a connected control module via the module interface provided by the respective module connector, and to control at least one infusion pump connected with the base module in dependence on that control information.

This is based on the idea to segregate resources (e.g. hardware, software, parameter values, formulae and/or mathematical models) from the base module which are specific for (or associated to) a given medical function. These resources are provided by means of a control module associated to the medical function. Control information specific for a given medical function may be provided to the base module by the control module, so that the base module is enabled to perform the medical function. Performance of a medical function by the base module may be conducted by controlling the infusion pump in dependence on the control information received from the control module associated with the medical function. Furthermore, a plurality of control modules may provide a plurality of medical functions. Therefore, the base module may be enabled to perform a plurality of different medical functions by connection with the respective control modules. At the same time, it is not necessary that the base module comprises all resources necessary for performing a medical function, because these resources are at least partially provided by the one or more control modules. A modification or an addition of a medical function to be performed by the base module does not necessarily require testing each possible combination of medical functions. Instead, it can be sufficient to test the modified or added medical function. The specific resources of the medical function are provided by a control module that communicates with the base module via the module interface (provided by the respective module connector) thereof. It may, therefore, be sufficient to test whether or not the control module comprising the specific resources of the modified or added medical function correctly communicates via the module interface. Even if all medical functions are new or modified, it may be sufficient to perform only a number of tests of the order of N. As a result, the base module is able to perform a plurality of medical functions while ensuring a reliable function of the base module in an efficient manner, i.e., with a reasonable test effort.

Depending on which medical function is to be performed by the base module, correspondingly, the associated control module can be selected and connected with a module connector of the base module. The base module may comprise, e.g., two, three, four, five or more module connectors. Each of the module connectors is connectable with one control module. Each of the module connectors provides a module interface serving as a shared boundary across which the base module and a connected control module can exchange information. The plurality of module connectors and module interfaces may be of the same kind. The base module may be connected with one or with more than one control module at the same time and can be able to perform different medical functions. Furthermore, the base module may be adapted to perform more than one medical function at the same time.

The module connectors may be designed as electrical and/or optical connectors that may be connectable with a mating connector of a control module.

The base module may comprise one or more, e.g. two, three or four, infusion pump connectors. Each infusion pump connector may be adapted to be releasably connected with one or more infusion pumps in order to operatively connect the base module with the infusion pump. As an example, the base module may control the infusion pump by providing control signals to the infusion pump via the infusion pump connector. The control signals may be generated by the base module based on the control information received from the control module. Alternatively, the control signals may be generated by the control module and forwarded by the base module to the infusion pump. In particular, the infusion pump connector may be different (and/or spaced) from the module connectors. It is also possible that the base module is firmly (i.e., not releasably) connected to the one or more infusion pumps. Furthermore, one or more infusion pumps may be integrated into the base module, e.g., be at least partially accommodated in a common housing.

According to an embodiment, the base module comprises at least one input device. The input device is adapted for receiving information which may particularly be associated with one or more medical functions (to be performed by the base module) and/or with one or more patients to be treated by the medical function. As an example, the base module may comprise a keyboard, a touchscreen, a computer mouse and/or a speech control as input device. The input device may be arranged at a housing of the base module; alternatively, the base module may comprise one or more (wired or wireless) connectors for a connection of external input devices. As another example, the base module may be connectable with a network via a corresponding connector and receive input information via the network (e.g. wired or wireless local area network, cellular network or others). Input information may be processed by the base module, may be provided to one or more control modules via the module interface of the corresponding module connector and/or may be provided to one or more infusion pumps. Input information for example comprises patient-related information, such as name, address, age, gender, weight, allergies, intolerances, patient history and the like and/or information related to the given medical function, such as start time and/or duration of the performance of the medical function (e.g. an infusion), target concentrations (e.g. of a substance in the patient's blood) and so forth, or other types of information. If the base module is connected with more than one control module, the base module may serve as a central unit. The base module may then serve for receiving input information associated with the plurality of medical functions via the input device.

Alternatively or in addition, the base module may comprise at least one output device. The output device is configured and provided for outputting information. The output information is for example associated with one or more medical functions and/or a patient. However, the output device may also output other types of information. As examples, the output device may be a display, a light source, a loudspeaker, a printer, a projector or the like. Alternatively, an output device may be provided in the form of a network connection, wherein the output device transmits information to the network (e.g. to a central storage). The output device may output information indicative for the status of a medical function, a health status of a patient and/or other types of information.

According to an embodiment, the base module comprises a computer that processes input information, output information, control information, control signals and/or other information. In particular, the base module may be designed as a programmable logic controller and/or comprise a computer. Programmable logic controllers may be reliable and flexible.

According to another aspect of the invention, a medical system for infusing liquids (or fluids in general) is provided. The liquids may particularly be infused into a circulatory system of a patient. The medical system comprises a base module being connected or connectable with at least one infusion pump such as to be able to control the infusion pump. It is provided that the base module is designed according to any embodiment of a base module disclosed herein. Furthermore, the medical system comprises at least one control module to be connected with the base module and being adapted to provide control information to the base module via the module interface provided by the module connector the control module is connected to.

The one or more control modules may each comprise resources being specific for a medical function. The base module can perform a medical function by controlling the infusion pump in dependence on the control information received by the respective control module. The resources associated with a specific medical function comprises, e.g., specific hardware, software, parameter values, formulae, mathematical models and/or others. In particular, the medical system may comprise a plurality of control modules. Each of the plurality of control modules may provide resources associated with a medical function.

According to another embodiment, one control module comprises resources associated with a plurality of medical functions. By connecting the control module with the base module, the latter may then be able to perform said plurality of medical functions by receiving the corresponding control information from the control module and controlling one or more infusion pumps accordingly.

The medical function may be a continuous infusion, an intermittent infusion, a patient-controlled infusion and/or more complex types of infusion. In particular, the medical function may be a type of a target-controlled infusion or a glycaemia control. Therein, a certain result is to be achieved, such as to reach or maintain a certain concentration of a substance in the blood or blood plasma of a patient (e.g. for anaesthesia or for treating diabetes). Resources that correspond to a target-controlled infusion may, e.g. and among others, include a mathematical model for calculating an estimated concentration of a substance in the blood/blood plasma and/or parameter values. As an example, the medical function may be a target-controlled infusion as described in DE 103 35 236 B3.

The control module may provide a user interface, in particular a graphical user interface, via the module interface provided by the module connector of the base module the control module is connected to. The base module may then reproduce the user interface by means of input and/or output devices, such as a display, a touchscreen, a speech control, a keyboard, a computer mouse, a loudspeaker, a light source and the like. Inputs made by a user at the input device(s) of the base module may be transmitted to the control module providing the user interface.

In order to perform a certain medical function, it may be necessary to acquire certain actual data (such as an ambient temperature and/or atmospheric pressure), in particular patient-related data, such as a blood pressure, a heart rate, a concentration of substances in the blood/blood plasma, a body temperature, body weight and the like. The one or more control modules may comprise at least one sensor being capable to acquire such data. Such a sensor may be connected or connectable with the control module, e.g. via a serial, Ethernet, Wi-Fi and/or Bluetooth communication link, and provide sensor data to the control module. The control module may then generate the control information based on at least part of the sensor data and provide the control information to the base module.

The control module may be designed as a module for a programmable logic controller. As the base module may be a programmable logic controller, the (at least one) control module may be connected with the base module via a connector of the programmable logic controller, the connector of the programmable logic controller being one of said plurality of module connectors. Programmable logic controllers may be reliable and flexible. In general, the control module and/or the base module may comprise a computer.

The control module may comprise a software storage device adapted for storing at least one piece of software, such as a software application. The software application may be adapted to generate control information and provide the same to the base module. The base module may then perform a medical function based on the control information. Therefore, the software application is a resource associated to the medical function.

Furthermore, the control module may comprise a processor. The processor is particularly adapted for processing at least one software application of the control module. While being processed by the processor, the software application may take into account sensor data from one or more sensors connected with the control module.

The object is also achieved by a method for testing a medical function to be performed by a base module for controlling infusion pumps. According to the method, in a first step a control module is provided comprising resources specific for the medical function to be tested as well as a base module connector. Therein, the control module is connectable with a module connector of the base module by means of the base module connector, wherein the module connector of the base module provides a module interface. Further, the control module is adapted to provide control information to the base module via the module interface provided by the module connector thereof and the base module performs the medical function by controlling at least one infusion pump based on the control information. In a second step, the base module connector of the control module is connected with a module connector of a test device such as to enable a communication between the control module and said test device via the module connector thereof, the module connector providing a module interface. In a third step, a set of rules is defined for the communication between the control module and the test device. A set of rules may correspond to all possible or allowed commands and responses communicated via the module interface of the test device. In a fourth step, it is tested by means of the test device whether or not the communication between the control module and the test device via the module interface provided by the module connector complies with the set of rules defined for the communication between the control module and the test device (according to the third step).

In this way the resources specific for the medical function (comprised by the corresponding control module) may be tested in order to ensure the correct performance of the associated medical function without the need of a test of other components, i.e., in an efficient manner. The resources may comprise hardware, software, parameter values, formulae, mathematical models and/or others. The test method may be a so-called regression test.

The module interface of the test device may be of the same design as the module interface of the base module. As an alternative, the base module itself may comprise means for testing a connected control module and therefore act as a test device.

It will be appreciated that the third step (defining the set of rules) of the method may be performed at any time before the fourth step (testing the compliance with the set of rules); e.g., the set of rules may be defined before connecting the control module with the test device during the test. The set of rules may also be used for multiple tests.

The advantages and advantageous embodiments described above with regard to the base module and medical system equally apply also to the method, such that it shall be referred to the above.

The idea underlying the invention shall subsequently be described in more detail with regard to the embodiments shown in the figures. Therein:
- Fig. 1A: shows a schematic view of an embodiment of a medical system comprising an embodiment of a base module and a plurality of control modules;
- Fig. 1B: shows the medical system of Fig. 1A, wherein the base module is connected with the control modules and the infusion pump via cables;
- Fig. 2: shows a schematic view of another embodiment of a medical system comprising another embodiment of a base module and a plurality of control modules;
- Fig. 3: shows a flow diagram of a method for testing a medical function to be performed by a base module; and
- Fig. 4: shows a test setup for testing a medical function.

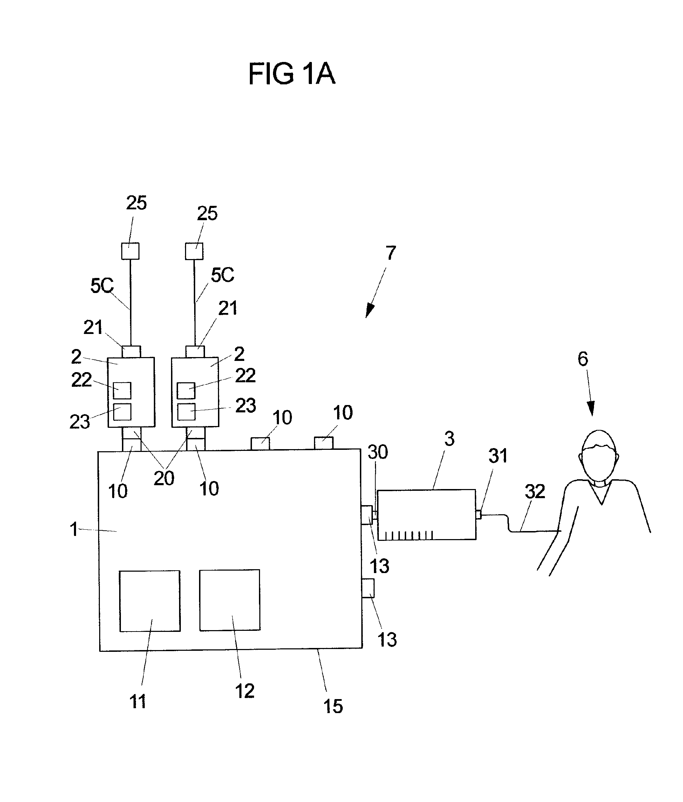

Fig. 1A is a schematic representation of a medical system 7 comprising a base module 1, several control modules 2 and an infusion pump 3. The medical system serves for infusing liquids, in particular into a circulatory system of a patient 6.

The base module 1 is operatively connected with the infusion pump 3 via one of a plurality of (presently two) infusion pump connectors 13 of the base module 1 and a base module connector 30 of the infusion pump 3. The base module 1 controls the infusion pump 3, e.g. by sending control signals in a suitable manner. In addition, the base module 1 may also receive information from the infusion pump 3 (such as status information, information regarding the rate and/or amount of infused liquid and the like).

The infusion pump 3 may be any suitable type of an infusion pump. In the present example, the infusion pump 3 comprises an actuator (not shown), such as an electric motor. By operating the actuator, a syringe may be operated in order to press a liquid contained in the syringe out of the syringe. The syringe may be a part of the infusion pump 3 or it may be connectable with the infusion pump 3 (and, e.g., disposable). The infusion pump 3 in general and in particular the syringe of the infusion pump 3 has an infusion outlet 31 connectable with one end of a hose 32. On its other end, the hose 32 is connected or connectable with an injection needle (not shown in the figure) to be applied on the patient 6 in any suitable manner.

The base module 1 further comprises an output device in the form of a display 11 for displaying information. For example, the display 11 may show information related to the progress of the infusion, patient-related information, alerts and/or other information. In addition, the base module 1 comprises an input device in the form of a keyboard 12. The keyboard 12 may be operated by a user (e.g. a physician, a nurse or the patient) in order to set up the medical system, and start, stop and/or supervise the infusion. Of course the base module 1 may also comprise more than one input and/or output devices and in particular also other types of input/output devices.

The base module 1 further comprises a plurality of presently four module connectors 10. Each module connector 10 serves for (releasably) connecting to a control module 2 and provides a module interface serving as a shared boundary across which the base module 1 and a connected control module 2 can exchange information. In the example shown in Fig. 1A, two module connectors 10 are connected with a control module 2 each, while two other module connectors 10 are not connected to a control module 2. A connection of a control module 2 with the base module 1 via a module connector 10 enables a communication between the respective control module 2 and the base module 1. In the present example, the communication is bidirectional, while other embodiments with a unidirectional communication are also conceivable. In the medical system 7 according to Fig. 1A a connection of the control modules 2 with the base module 1 is established by means of a base module connector 20 of each of the control modules 2. The base module connectors 20 are connected with the module connectors 10, which could be formed as mating electrical and/or optical connectors. In particular, the control modules 2 could also be formed as cassettes, which are inserted at the base module 1 and thereby plugged on the module connector 10 for a connection.

Each control module 2 connected with the base module 1 provides control information to the base module 1 via the module interface provided by the connected module connector 10. The control information is used by the base module 1 for controlling the infusion pump 3. That is, the base module 1 controls the injection of liquid in the circulatory system of the patient 6 based on the control information received by at least one of the control modules 2. Thereby, the base module 1 performs a medical function.

Examples of medical functions are a continuous infusion, an intermittent infusion, a patient-controlled infusion and/or more complex types of infusion. The infusion rate may be controlled to follow a curve over the elapsed time. The curve may be predefined or may be calculated taking into account actual data. For example, patient-related information, such as age, gender, weight, blood pressure, heart rate, the concentration of substances in the blood, body temperature and the like may be taken into account. In particular, the medical function may be a type of a target-controlled infusion. Different types of target-controlled infusion are, e.g., the Mash model, the Schnider model and variants of these models for obese and/or elder patients or children. Further, the medical function may be a glycaemia control.

Each control module 2 comprises the resources that are specific to this medical function in order to perform the medical function. Of course, various resources may be specific to more than one medical function. The resources may comprise hardware, software, parameter values, formulae, mathematical models and the like.

The control modules 2 according to Fig. 1A each comprise software associated to the medical function associated to the control module 2 and a storage device 22 for storing that software. Each control module 2 further comprises a processor 23 for processing the software associated to the medical function.

Moreover, each of the control modules 2 shown in Fig. 1A comprises a sensor 25. The sensors 25 are connected with the control modules 2 by means of a sensor connector 21 and via a cable 5C. The sensors 25 are adapted for measuring at least one certain quantity and provide sensor data indicative for the quantity to the connected control module 2. The sensors 25 may be adapted to determine a quantity associated with the medical function (e.g. a target-controlled infusion or glycaemia control function) provided by the respective control module 2. For example, the sensors 25 may determine concentrations of given substances in the blood/blood plasma or breath of the patient 6 and/or variables of certain body functions, such as heart rate, blood pressure, body temperature, etc. Of course, each control module 2 may comprise more than one sensor 25 and/or more than one sensor connector 21, for example when the associated medical function is based on more than one quantity. On the other hand, a control module may not comprise any sensor 25.

Sensor data from a sensor 25 received by one of the control modules 2 is forwarded to the base module 1 as control information and/or is processed by the control module 2. In particular, the software of the control module 2 may process the sensor data and provide control information to the base module 1 based on the sensor data. As an example, a sensor 25 may measure a concentration of an infused substance in the blood of the patient 6. Depending on the measured concentration, the control module 2 sends control information to the base module 1. In this case or in general, control information may comprise commands or signals for controlling the infusion pump 3. The base module 1 then controls the infusion pump 3 accordingly by providing control signals.

As explained before, the base module 1 comprises output and input devices (in the present example in the form of a display 11 and a keyboard 12). These devices are also used to implement a human machine interface, in particular a GUI (graphical user interface). On the one hand, a GUI may be provided by the base module 1 itself. On the other hand, the base module 1 may serve to transmit data from and to the display 11 and keyboard 12 (i.e. to publish the GUI), while the GUI is provided by a control module 2. Each control module 2 may provide a GUI and the base module 1 allows to select which GUI shall be published by the output and input devices (i.e., the display 11 and the keyboard 12). The data for the GUI is communicated via the module interface provided by the module connector 10 of the base module 1. The control modules 2 therefore do not comprise a display and/or a keyboard or touchscreen. Eventually, the control modules 2 even do not need to comprise any input/output devices for user interaction or for reproduction of a user interface. Thus, the control modules 2 of the present example are not autonomous.

Communication via the module interface provided by the module connector 10 may comprise messages, user interface data, infusion pump flow rates, infusion pump statuses, user inputs, sensor data, commands, infusion pump control signals and others. Moreover, different control modules 2 may also communicate with each other via the base module 1. As an example, the control modules 2 may exchange sensor data, so that redundant sensors may be obsolete.

By providing the resources specific for a certain medical function segregated in the control modules 2, the medical function may be developed independently of the resources of other medical functions. When a user wants to perform a certain medical function, he simply needs to connect the corresponding control module 2 with the base module. Therefore, the medical system 7 is easily extendable by adding further control modules 2. The medical system 7 is, therefore, a modular system.

The base module 1 comprises four module connectors 10. Therefore, up to four control modules 2 may be connected with the base module 1 at the same time. If more than four control modules 2 are available, the user may connect only those control modules 2 which comprise the resources specific for the medical function(s) he wants to perform.

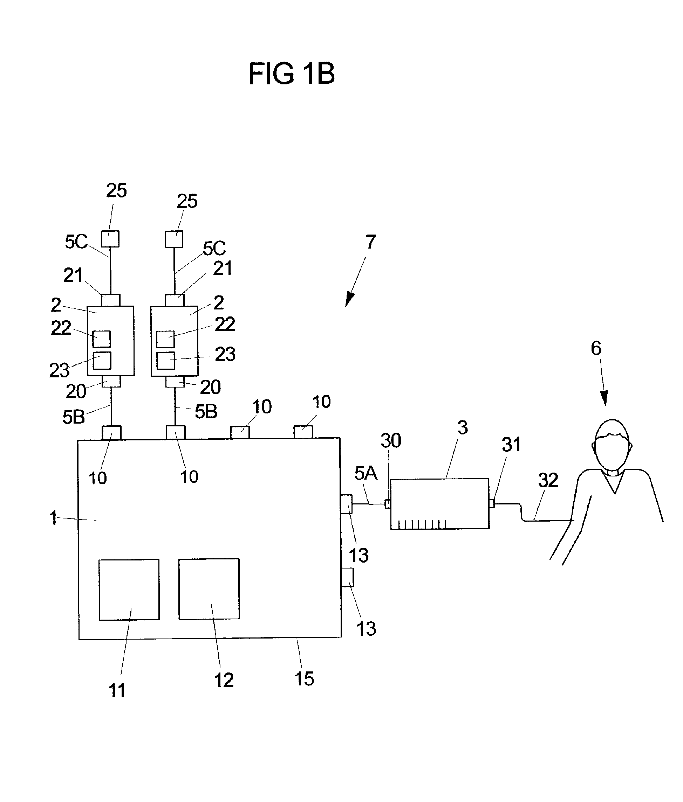

Fig. 1B shows the medical system 7 according to Fig. 1A with the difference that according to Fig. 1B the base module connector 20 of each control module 2 is connected with a module connector 10 of the base module 1 by means of a cable 5B. Furthermore, also the base module connector 30 of the infusion pump 3 is connected with an infusion pump connector 13 of the base module 1 by means of a cable 5A. The cables 5A, 5B could for example be electrical cables, particularly with a plurality of separate conductors, and/or optical cables. By use of cables, the connected components could be arranged spaced to one another, while, on the other hand, a direct connection without cables may allow for a more compact design of the medical system 7.

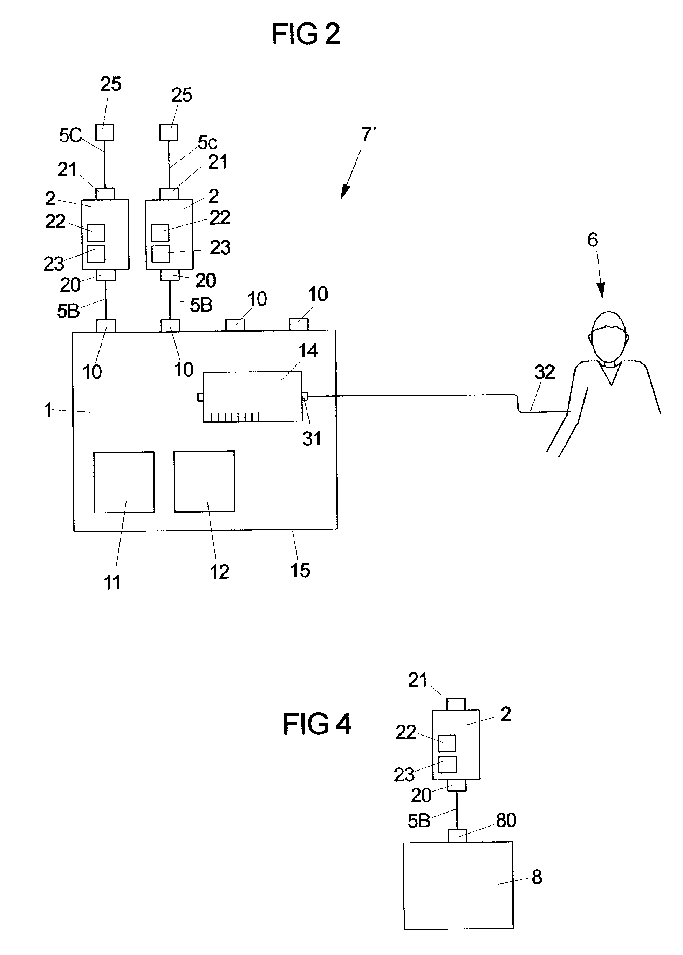

Fig. 2 shows another embodiment of a medical system 7' which differs from the medical system 7 according to Fig. 1B in that the base module 1' according to Fig. 2 is not connected with an external infusion pump via a releasable connection, but comprises an infusion pump 14, that is accommodated in a common housing 15 of the base module 1'. For the other components and functions of the medical system 7', reference is made to the corresponding description of Fig. 1A, 1B.

An advantage of the medical systems 7, 7' according to Fig. 1A, 1B and 2 is, that new medical functions can be added to the medical system 7, 7' in a simple manner. Also, existing medical functions may be updated or modified in a simple manner by updating or modifying the associated resources of the corresponding control module 2. Both when adding and modifying a medical function, it is not necessary to perform tests on the complete medical system 7, 7' what could be time-consuming and cumbersome. Instead, it is sufficient to test the control module comprising the resources of the new/modified medical function. As a result, the medical system 7, 7' allows to perform a plurality of medical functions while efficiently ensuring that the infusion pump 3 is controlled correctly.

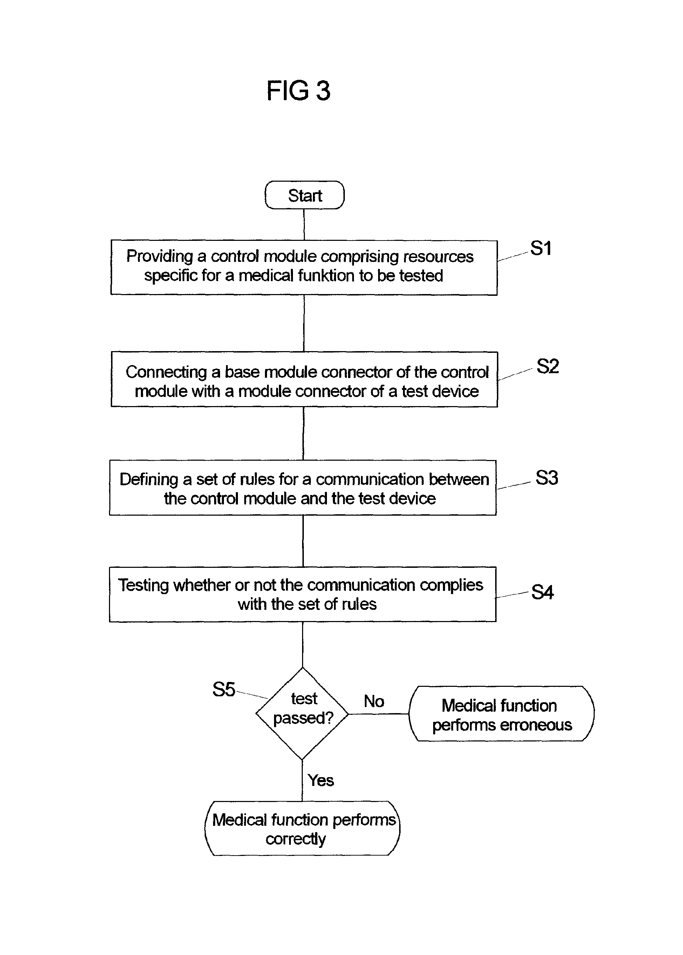

A method for testing a new or modified medical function is now described with reference to Fig. 3. Regarding the medical system 7, 7', the method corresponds to a so-called regression test.

In a first step S1 a control module 2 is provided comprising all resources specific for the medical function to be tested. The control module 2 has already been described with reference to Fig. 1A, 1B and 2.

In a second step S2, the base module connector 20 of the control module 2 is connected with a test device 8 such as to enable a communication between the control module 2 and the test device 8.

This test setup is shown in Fig. 4, wherein the base module connector 20 of the control module 2 is connected with a module connector 80 of the test device 8 by means of the cable 5B. The module connector 80 of the test device 8 provides a module interface which is of the same kind as the module interface provided by the base module 1,1' described with reference to Fig. 1A, 1B and 2. The module connector 80 of the test device 8 may also be designed so as to allow for a direct plug connection with the base module connector 20 of the control module 2 without an intermediate cable.

Turning back to Fig. 3, a third step S3 of the test method is now described. Therein, a set of rules for the communication between the control module 2 and the test device 8 is being defined. The set of rules corresponds to all possible or allowed types of communication, e.g. commands and responses, transmitted between the control module 2 and the test device 8 via the module interface provided by the module connector 80 of the test device 8. The set of rules may be a sort of a catalogue.

In a fourth step S4, the communication between the control module 2 and the test device 8 is tested, e.g., by transmitting all possible commands to the control module 2 by means of the test device 8 and analysing all responses from the control module 2. By comparing the responses with the set of rules, the test device 8 indicates whether or not the control module 2 complies with the set of rules. The test device 8 may comprise suitable means for an indication of the test result. Of course, step three S3 may be performed at any time before step four S4.

Finally, at a fifth step S5, a decision is made whether the tested medical function performs erroneous or correctly.

As an alternative, the base module 1,1' may be adapted to be able to perform the test described above, i.e., comprise suitable means such that it may be used as a test device. In this way the testing procedure may be further simplified as no additional test device is needed.

The idea underlying the invention is not limited to the embodiments described above, but may be implemented also in entirely different embodiments.

In particular, the base module 1,1' may comprise or be connectable to more than one or two infusion pumps 3, 14. For example, the base module 1,1' may comprise or be connectable to three, four or five infusion pumps 3, 14. The base module 1,1' may be able to control more than one infusion pump 3, 14 at the same time. Therefore, the base module 1,1' may perform a plurality of medical functions at the same time, eventually at more than one patient 6.

Furthermore, the medical system 7, 7' may comprise more control modules 2 than can be connected with the base module 1, 1' at the same time. For example, the base module 1,1' comprises four module connectors 10 and the medical system 7, 7' could comprise six control modules 2. Depending on the medical functions to be actually performed, a selection of the six control modules is connected with the base module 1, 1'.

Instead of using cables 5A-5C, the connections between the control modules 2, the base modules 1, 1', the sensors 25 and/or the infusion pumps 3, 14 may be established wirelessly or with direct plug connections.

### List of reference numerals

- 1, 1': Base module
- 10: Module connector
- 11: Display
- 12: Keyboard
- 13: Infusion pump connector
- 14: Infusion pump
- 15: Housing
- 2: Control module
- 20: Base module connector
- 21: Sensor connector
- 22: Software storage device
- 23: Processor
- 25: Sensor
- 3: Infusion pump
- 30: Base module connector
- 31: Infusion outlet
- 32: Hose
- 5A-5C: Cable
- 6: Patient
- 7, 7': Medical system
- 8: Test device
- 80: Module connector

## Claims

1. A base module for controlling infusion pumps, being connected or connectable with at least one infusion pump for infusing liquids, in particular into a circulatory system of a patient,
**characterized by**
a plurality of module connectors (10) via each of which the base module (1, 1') is connectable with an external control module (2), wherein each of the module connectors (10) provides a module interface, wherein the base module (1, 1') is adapted to
- receive control information from a control module (2) via the module interface provided by the module connector (10) the control module (2) is connected to and
- control at least one infusion pump (14, 3) connected with the base module (1, 1') in dependence on the received control information.

2. The base module according to claim 1, **characterized in that** each of the module connectors (10) is designed as an electrical or optical connector.

3. The base module according to any of the preceding claims, **characterized in that** the base module (1) includes at least one infusion pump connector (13) for operatively connecting at least one infusion pump (3).

4. The base module according to any of the preceding claims, **characterized by** an input device (12) adapted for receiving input information associated with at least one medical function performed by the base module (1, 1') and/or at least one patient (6).

5. The base module according to any of the preceding claims, **characterized by** an output device (11) adapted for outputting output information associated with at least one medical function and/or at least one patient (6).

6. The base module according to any of the preceding claims, **characterized in that** the base module (1, 1') is designed as a programmable logic controller.

7. A medical system for infusing liquids, in particular into a circulatory system of a patient, comprising a base module for controlling infusion pumps, the base module being connected or connectable with at least one infusion pump,
**characterized in**
**that**
- the base module is a base module (1, 1') according to any of the preceding claims and
- the medical system (7, 7') comprises at least one control module (2) adapted to provide control information to the base module (1, 1') via the module interface provided by the module connector (10) of the base module (1, 1') when the control module (2) is connected to one of the plurality of module connectors (10).

8. The medical system according to claim 7, **characterized in that** the at least one control module (2) comprises resources being associated with a medical function, wherein the base module (1, 1') is configured to perform a medical function by controlling the infusion pump (14, 3) in dependence on the control information received by the control module (2).

9. The medical system according to claim 8, **characterized in that** the medical function is a target-controlled infusion function or a glycaemia control function.

10. The medical system according to any of claims 7 to 9, **characterized in that** the control module (2) is adapted to provide a user interface via the module interface provided by the module connector (10) of the base module (1, 1') the control module (2) is connected to.

11. The medical system according to any of claims 7 to 10, **characterized in that** the control module (2) is connected to or connectable with a sensor (25) for receiving sensor data provided by the sensor (25), wherein the control information provided by the control module (2) to the base module (1, 1') is generated by the control module (2) in dependence of at least a portion of the sensor data.

12. The medical system according to any of claims 7 to 11, **characterized in that** the control module (2) is designed as a module for a programmable logic controller.

13. The medical system according to any of claims 7 to 12, **characterized in that** the at least one control module (2) comprises a software storage device (22) storing at least one software application associated with the medical function performed by the base module (1, 1'), the software application providing control information to the base module (1, 1').

14. The medical system according to any of claims 7 to 13, **characterized in that** the control module (2) comprises a processor (23) adapted for processing the software application of the control module (2).

15. A method for testing a medical function to be performed by a base module for controlling infusion pumps, **characterized by**
- providing (S1) a control module (2) comprising resources specific for the medical function to be tested and a base module connector (20),
o wherein the control module (2) is connectable with a module connector (10) of the base module (1, 1') via the base module connector (20), wherein the module connector (10) of the base module (1, 1') provides a module interface, and
o wherein the control module (2) is adapted to provide control information to the base module (1, 1') via the module interface provided by the module connector (10) thereof, wherein the base module (1, 1') performs the medical function by controlling at least one infusion pump (14, 3) in dependence on the control information;
- connecting (S2) the base module connector (20) of the control module (2) with a module connector (80) of a test device (8) to enable a communication between the control module (2) and the test device (8) via the module connector (80) of the test device (8), the module connector (80) providing a module interface;
- defining (S3) a set of rules for the communication between the control module (2) and the test device (8);
- testing (S4) whether or not the communication between the control module (2) and the test device (8) via the module interface provided by the module connector (80) complies with the set of rules defined for the communication between the control module (2) and the test device (8).
